# EUROPEAN PATENT SPECIFICATION

(11) **EP 2 640 827 B1**
(45) Date of publication and mention of the grant of the patent: **19.07.2023**
(21) Application number: 11840811.1
(22) Date of filing: 16.11.2011
(51) Int. Cl.: C12N 1/02, C12N 1/04

(54) **METHODS FOR PRESERVING TARGET CELLS**
VERFAHREN ZUR KONSERVIERUNG VON ZIELZELLEN
PROCÉDÉS DE CONSERVATION DE CELLULES CIBLES

(30) Priority: 16.11.2010 US 414335 P
(43) Date of publication of application: 25.09.2013
(73) Proprietor: Ingeneron, Inc., Houston, TX 77054 (US)
(72) Inventor: COLEMAN, Michael, Houston, TX 77381 (US); VYKOUKAL, Jody, Houston, TX 77098 (US)
(74) Representative: Weickmann & Weickmann PartmbB
(86) International application number: PCT/US2011/060990
(87) International publication number: WO 2012/068248

(56) References cited:
- WO-A1-82/00660
- WO-A1-2007/129828
- DE-A1- 4 341 005
- US-A1- 2007 042 341
- US-A1- 2010 239 671
- US-B1- 6 184 011

## Description

### TECHNICAL FIELD

This invention relates to methods for preserving target cells from a fluid, cell-containing sample, and more particularly to preserving target cells from a fluid, cell-containing sample using a matrix that retains target cells and that can be degraded before or after cryopreservation to recover the target cells.

### SUMMARY

This document is based on the discovery of a method for preserving target cells using a degradable three-dimensional matrix. The three-dimensional structure of the matrix provides a large surface area for capture of the target cells. The matrix can be preserved (e.g., cryopreserved using a solution containing dimethylsulfoxide). When target cells are needed, the matrix can be degraded and the target cells retained by the matrix can be recovered. Cells recovered using the methods described herein can be used for tissue culture, diagnostic testing, further purification, or therapeutic administration.

The invention relates to a method for obtaining target cells from a fluid, cell-containing sample. The method includes providing a fluid, cell-containing sample from a subject; passing the sample through a three-dimensional matrix, the matrix comprising an inner core and an outer layer disposed around the inner core, the inner core comprising a non-degradable substrate, the outer layer composed of a degradable polymer and wherein the outer layer comprises a capture ligand attached thereto, wherein the capture ligand has affinity for a target cell in the sample; recovering target cells retained by the matrix by substantially degrading the outer layer of the matrix with a degradative enzyme. The capture ligand has affinity for a target cell in the sample as described herein. The inner core can be composed of woven or non- woven polypropylene, nylon, silk mesh, or fabric. In any of the methods described herein, the fluid, cell-containing sample can include erythrocytes, and the method can include removing erythrocytes from the fluid, cell-containing sample to produce an erythrocyte depleted sample before passing the erythrocyte depleted sample over a degradable three-dimensional matrix.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, the exemplary methods and materials are described below. In case of conflict, the present application, including definitions, will control. The materials, methods, and examples are illustrative only and not intended to be limiting.

Other features and advantages of the invention will be apparent from the following detailed description, and from the claims.

US 6 184 011 B1 discloses a method for obtaining target cells from a fluid, cell-containing sample, the method comprising:
a) providing a fluid, cell-containing sample (e.g. E. Coli suspension in example 1);
b) passing the sample through a degradable, three-dimensional matrix (in a capture cartridge with CDB-IC cellulose matrix), and comprising a capture ligand attached thereto, wherein the capture ligand (CDB antibody) has affinity for a target cell in the sample; and
c) recovering target cells retained by said matrix by substantially degrading the matrix by contacting the matrix with a degradative enzyme (cellulase).

DE 43 41 005 A1 discloses a method for obtaining white blood cells from a fluid, cell-containing sample also containing red blood cells, the method comprising:
a) providing a fluid, cell-containing sample;
b) passing the sample through a three-dimensional matrix, wherein the matrix comprises an inner core and an outer layer disposed around the inner core, the inner core comprising a nondegradable substrate (plant cell walls, which are non-degradable with respect to the enzyme collagenase), the outer layer composed of collagen; and
c) recovering target cells retained by said matrix by substantially degrading the outer layer of the matrix by contacting the matrix with collagenase.

### DESCRIPTION OF DRAWINGS

**FIG. 1A** is a schematic diagram of two types of a matrix. The left panel is a representation of a two dimensional section through a three dimensional matrix having a honeycomb pattern. The right panel is a representation of a two dimensional section through a three dimensional matrix having a grid-like pattern.
**FIG. 1B** is a schematic diagram of a method of obtaining target cells using a three dimensional matrix within a conical tube.
**FIG. 2** is a graph of the percentage of Ramos tumor cells trapped using the following collagen matrices: BIOSTEP, CM Sponge, FIBRACOL, PROMOGRAN, and SkinTemp.
**FIG. 3A** and **3B** are graphs of the percentage of Ramos cells trapped on Puracol MicroScaffold^{™} collagen.
**FIG. 4** is a graph of the amount of fluorescence as a function of collagenase concentration over different incubation periods (0 min, 4 min, 10 min, 30 min, 1 hr, 2 hr, or 24 hr) for gelatin beads (Cultispher) loaded with fluorescein.

### DETAILED DESCRIPTION

In general, this document is based on an affordable point-of-use platform for the rapid isolation of target cells from fluid, cell-containing samples. As used herein, "fluid, cell-containing sample" refers to a liquid containing a suspension of cells. Non-limiting examples of such fluid, cell-containing samples include blood samples (e.g., peripheral blood or umbilical cord blood), bone marrow aspirates, lymph, cerebral spinal fluid, the liquid fraction of a lipoaspirate, ductal fluid, or needle biopsy aspirates. Such fluid, cell-containing samples can be obtained from any mammalian subject, including humans, monkeys, mice, rats, rabbits, guinea pigs, dogs, or cats. In some embodiments, the cells from a sample such as blood sample can be washed (e.g., with phosphate buffered saline) and resuspended in saline, physiological buffer, or culture medium before processing as described herein. In some embodiments in which the fluid, cell-containing sample contains erythrocytes, the erythrocytes can be removed by, for example, density gradient sedimentation or hetastarch aggregation.

Target cells retained by the matrix are viable and can be used for any purpose, including tissue culture, characterization, diagnostic testing, or further purification. In some embodiments, matrices containing target cells are frozen and used, for example, for cell banking of umbilical cord blood or other cell-containing samples, or for banking of stem or other target cells. Target cells found in fluid, cell-containing samples can include, for example, fetal blood cells, white blood cells, circulating tumor cells, disseminated tumor cells, stem cells, or bacteria (e.g., Staphylococcus or Streptococcus). For example, in some embodiments, fetal blood cells can be recovered from a sample of maternal blood and used for non-invasive prenatal diagnosis. Stem cells can be recovered, for example, from a sample of umbilical cord blood. Circulating tumor cells or disseminated tumor cells can be recovered from a fluid, cell-containing sample (e.g., peripheral blood, bone marrow, or lymph) to detect metastasis in a patient, determine prognosis in patients, or test for drug resistance. Bacteria can be removed from a fluid, cell-containing sample (e.g., peripheral blood sample) to detect sepsis.

### Three Dimensional Matrices

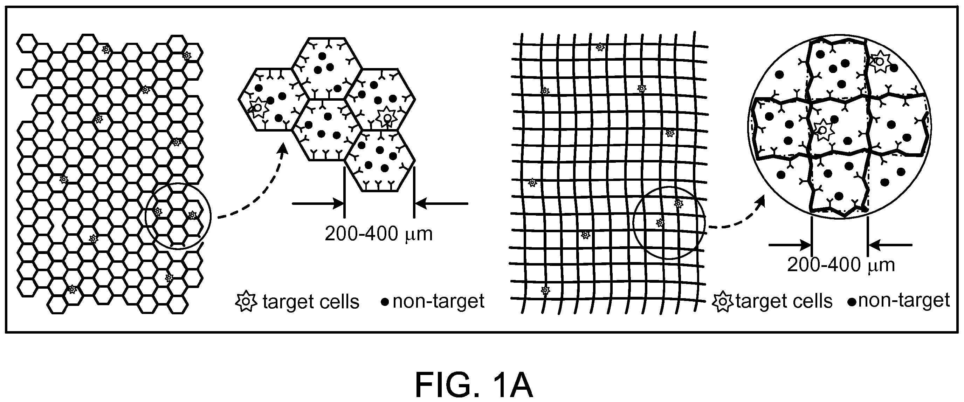

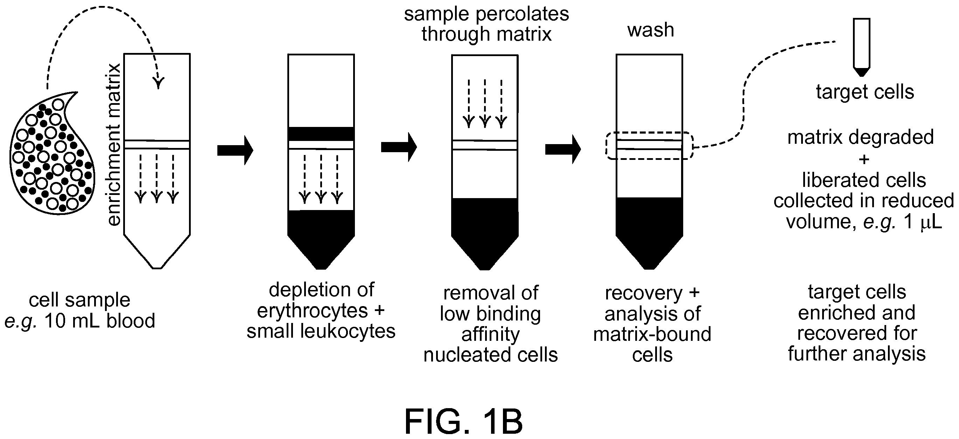

The structure of a matrix for use in a method of the invention is defined in the appended claims. Other types of matrices are described herein for comparative purposes. For suitable three dimensional matrices described herein, no dimension of the matrix is less than 0.5 µM, providing a large surface area for capture of target cells. A three dimensional matrix can have a porosity (e.g., <10 µM) that retains cells of a certain size (e.g., white blood cells) while allowing others (e.g., erythrocytes and platelets) to pass through the matrix. See, for example, FIG. 1A for a schematic representation of a two dimensional section through a three dimensional matrix having either a honeycomb or a grid-like pattern.

According to the invention, the outer layer of a three dimensional matrix contains a capture ligand attached thereto, where the capture ligand has affinity for a target cell in the sample. The capture ligand can be indirectly attached to the three dimensional matrix via, for example, avidin or streptavidin. For example, a three dimensional matrix can be functionalized with avidin or streptavidin, and a capture ligand can be biotinylated.

A capture ligand can have binding affinity for one or more cell surface molecules on a tumor cell. A cell surface molecule can be a cell adhesion molecule. In some embodiments, the cell surface molecule is epithelial cell adhesion molecule (EpCAM), mucin 1 (MUC1), human epidermal growth factor receptor 2 (HER2), or Melanoma-associated chondroitin sulfate proteoglycan (MCSP).

A capture ligand also can have binding affinity for one or more cell surface molecules (e.g., two different cell surface molecules) on a fetal blood cell. For example, a capture ligand can have binding affinity for one or more of CD71, CD36, CD45, glycophorin A, CD35, and CD47. Such cell surface molecules are found on fetal erythroblasts. See, for example, Ho et ah, Ann Acad Med Singapore 32:597-604 (2003). In some embodiments, a capture ligand has binding affinity for SSEA-4, CD 117, or CD 146.

A capture ligand can be an antibody or antigen-binding fragment thereof that has binding affinity for a target cell in the sample. In some embodiments, a suitable antibody or antigen-binding fragment thereof also can have affinity for immunoglobulin (e.g., anti-IgG antibody). "Antibody" as the term is used herein refers to a protein that generally includes heavy chain polypeptides and light chain polypeptides. IgG, IgD, and IgE antibodies comprise two heavy chain polypeptides and two light chain polypeptides. IgA antibodies comprise two or four of each chain and IgM generally comprise 10 of each chain. Single domain antibodies having one heavy chain and one light chain and heavy chain antibodies devoid of light chains are also contemplated. A given antibody comprises one of five types of heavy chains, called alpha, delta, epsilon, gamma and mu, the categorization of which is based on the amino acid sequence of the heavy chain constant region. These different types of heavy chains give rise to five classes of antibodies, IgA (including IgAl and IgA2), IgD, IgE, IgG (IgGI, IgG2, IgG3 and IgG4) and IgM, respectively. A given antibody also comprises one of two types of light chains, called kappa or lambda, the categorization of which is based on the amino acid sequence of the light chain constant domains.

"Antigen binding fragment" refers to an antigen binding molecule that is not an antibody as defined above, but that still retains at least one antigen binding site. Antibody fragments often comprise a cleaved portion of a whole antibody, although the term is not limited to such cleaved fragments. Antigen binding fragments can include, for example, a Fab, F(ab')2, Fv, and single chain Fv (scFv) fragment. An scFv fragment is a single polypeptide chain that includes both the heavy and light chain variable regions of the antibody from which the scFv is derived. Other suitable antibodies or antigen binding fragments include linear antibodies, multispecific antibody fragments such as bispecific, trispecific, and multispecific antibodies (e.g., diabodies (Poljak Structure 2(12): 1 121-1 123 (1994); Hudson et ah, J. Immunol. Methods 23(1-2): 177-189 (1994)), triabodies, tetrabodies), minibodies, chelating recombinant antibodies, intrabodies (Huston et ah, Hum. Antibodies 10(3-4): 127-142 (2001); Wheeler et al, Mol. Ther. 8(3):355-366 (2003); Stocks Drug Discov. Today 9(22): 960-966 (2004)), nanobodies, small modular immunopharmaceuticals (SMIP), binding-domain immunoglobulin fusion proteins, camelid antibodies, camelized antibodies, and VHH containing antibodies.

A capture ligand also can be a lectin having binding affinity for a sugar moiety that is on a glycoprotein or glycolipid. For example, the lectin can be soybean agglutinin (SBA), peanut agglutinin (PNA), Erythrina cristagalli lectin (ECL), Allomyrina dichotoma lectin (Alio A), Viscum album agglutinin (VAA), concanavalin A (Con A), Lens culinaris lectin (LcH), or Pisum sativum agglutin (PSA). SBA, PNA, ECL, Alio A, and VAA have binding affinity for galactose moieties while ConA, LcH, and PSA have binding affinity for glucose moieties.

Materials that can be used to fabricate the outer layer of a three dimensional matrix for use in the methods described herein can be generally categorized into two types: naturally derived materials (e.g., polymers) and synthetic materials (e.g., polymers). Non- limiting examples of naturally derived polymers include extracellular matrix (ECM) molecules (e.g., collagens, hyaluronic acid, or laminins) or products thereof (e.g., gelatin, which is partially hydrolyzed collagen), and polysaccharides (e.g., alginate, chitin, chitosan, agarose, or cellulose). Any such matrix, and blends of these materials with other polymers or other materials, is contemplated for use in the methods described herein. In one embodiment, the outer layer or the three dimensional matrix is a laminin rich gel.

Type I collagen, the most prevalent ECM molecule in the body, is readily isolated from animal tissues, or can be produced using recombinant DNA technology, and can be processed into a wide variety of structures for use in the methods described herein. For example, collagen can be woven into a three-dimensional framework such as a collagen sponge. Three dimensional matrices with a sponge-like structure also can be produced through lyophilization of collagen solutions, including reproducibly adjusting the mean pore size and geometry of such collagen sponges according to freeze-drying parameters. O'Briena et al, Biomaterials, 6: 1077-1086 (2004). As a biopolymer, collagen also is amenable to functionalization including with antibodies or other ligands using standard EDC (carbodiimide) / NHS mediated crosslinking approaches. See Kojima et al, Biomaterials 27(28): 4904 (2006). The structure and resultant mechanical properties of collagen-based matrices can be regulated by the process utilized to extract the collagen from tissues and by various crosslinking processes. Collagen molecules can be crosslinked physically by dehydrothermal or UV radiation treatments, or chemically by using various chemical agents. Suitable collagen matrices are described, for example, in U.S. Patent No. 5,885,829. A three dimensional matrix can be composed of one or more synthetic polymeric materials. Synthetic polymers can be processed with various techniques. The mechanical and physical properties of synthetic polymers can be readily adjusted through variation of molecular structures. Non-limiting examples of suitable synthetic polymers that are degradable include polyesters such as poly(glycolic acid) (PGA), poly(lactic acid) (PLA), poly(lactic acid)-poly(glycolic acid) (PLGA) polymers, polyhydroxybutyrate (PHB), or other polyhydroxyalkanoates. Further degradable matrices include polyanhydrides, polyorthoesters, and poly(amino acids). Any such matrix may be utilized to fabricate a three dimensional matrix having a structure that retains target cells or that can be derivatized. See, for example, U.S. Patent No. 5,885,829 for suitable synthetic polymer matrices.

According to the invention, a three-dimensional matrix contains an inner core and an outer layer disposed around the inner core. The outer layer is composed of a degradable polymer as described above and contains a capture ligand. The inner core is composed of a substrate that differs from the outer layer and is not-degradable in the same manner as the outer layer. For example, the inner core can be woven or non-woven polypropylene, nylon, silk mesh, or fabric.

In some embodiments, the three dimensional matrix is adapted in the form of an insert that can be fitted into a standard conical tube (e.g., a 15 or 50 mL centrifuge tube) See, for example, FIG. IB. After passing a fluid, cell-containing sample through the matrix within such a tube, the cells are trapped within the matrix while the non-target cells (e.g., red blood cells and platelets) are in the lower portion of the tube. The matrix can be further washed with a buffer. In some embodiments, a pump can be used to pass the sample through the matrix.

The matrix and target cells retained within the matrix can be cryopreserved using a solution containing dimethylsulfoxide (DMSO). For example, a solution containing 1 to 20% DMSO (e.g., 10% DMSO) can be used for cryopreservation. A solution containing DMSO and one or more reagents such as glycerol, 1,2-propanediol, dextran, trehalose, ethylene glycol, albumin, polyvinyl pyrolidone, or hydroxyethyl starch can be used to cryopreserve the matrix and target cells retained within the matrix. Serum (e.g., fetal bovine or human serum albumin) also can be used in combination with DMSO. After adding cryopreservative, the cells can be frozen (e.g., to -90°C). The matrix and cells retained within the matrix can be frozen at a controlled rate. The matrix and cells retained within the matrix can be frozen in a nonlinear rate. See, for example, U.S. Patent Application No. 20100240127. The frozen matrix and target cells within the matrix can be placed in the liquid phase of the liquid nitrogen storage tank for long term storage.

When it is desired to recover the target cells, the matrix can be thawed (e.g., in a 37°C water bath). Target cells can be recovered from a matrix by substantially degrading the matrix. As used herein, the term "substantially degrading" with reference to the matrix indicates at least 50% (e.g., at least 55%, 60%, 70%, 75%, 80%, 85%, or 90%) of the matrix has been degraded. For example, the matrix can be contacted with a degradative enzyme for an amount of time sufficient to degrade the matrix. Non-limiting examples of degradative enzymes include collagenase, hyaluronidase, proteases, chitosanase, alginate lyase, alginate depolymerase, and cellulase. For example, if the three dimensional matrix contains hyaluronic acid, the matrix can be degraded by contacting the matrix with a hyaluronidase for an amount of time sufficient to substantially degrade the matrix. If the degradable matrix is a protein a protease can be used to substantially degrade the matrix. Degrading the matrix can include contacting the matrix with an acidic or basic solution for an amount of time sufficient to substantially degrade the matrix.

The amount of time sufficient to substantially degrade the matrix can be determined empirically by one of ordinary skill in the art for the matrix employed. Factors such as type and concentration of enzyme, temperature and presence of chelation agents relative to required enzyme cofactors, and incubation time can be varied to determine the amount of time to degrade the matrix. After degrading the matrix, cells can be recovered using, for example, centrifugation.

In some embodiments, the cells can be recovered from the matrix and then cryopreserved as discussed above.

### Articles of Manufacture

This document also features articles of manufacture that include three dimensional matrices described herein. Three dimensional matrices can be combined with packaging material and sold as a kit. For example, a kit can include a three dimensional matrix derivatized with one or more ligands for capture of target cells (e.g., stem cells, fetal cells from maternal blood, or circulating tumor cells). A kit further can include one or more of an apparatus for sample collection such as a vacutainer blood collection tube and needle, a cryopreservative, culture medium, or reagents for characterizing the target cells. The packaging material included in a kit typically contains instructions or a label describing how the three dimensional matrix can be used to recover and/or preserve target cells from a fluid, cell-containing sample. Components and methods for producing such kits are well known.

The following are comparative examples outside the scope of the invention.

### EXAMPLES

### COMPARATIVE EXAMPLE 1

### Recovery of Cells Using a Degradable Matrix

In this experiment, a collagen sponge (Puracol^{™} Plus, 10 mm diameter) was used to recover white blood cells (WBC) from a test sample where the ratio of red blood cells (RBC) to WBC was 1000: 1. The collagen sponge was placed in a Swinnex 10 mm filter unit. The test sample was passed through the sponge, which then was gently rinsed by flushing with 3 mL of sterile phosphate buffered saline (PBS) at 200 µí̈/min. The collagen sponge then was removed from the filter unit and placed in a 35 mm culture dish containing 3 mL of sterile lactated Ringer's with 1.2 units/mL collagenase IV (CLS-4, Worthington Biochemicals, Lakewood, NJ). The sponge was incubated at 37°C with rotational shaking (approximately 60 rpm) for 30 min or until the sponge was digested. The contents of the culture dish were removed by pipette and placed into a 15 mL sterile conical centrifuge tube. Cells were recovered by centrifuging the tube at 400 x g for 5 min. Cells were washed 3x with 3 mL PBS to remove collagenase and then resuspended in desired buffer or media. Table 1 provides the estimated WBC recovery and RBC depletion factor at various dilutions, wash volumes, and flow rates.

**Table 1**

| blood volume, µL | ringers, µL | Dilution | wash volume, µL | flow rate µL/min | estimated WBC recovery | RBC:WBC on filter | RBC depletion factor |
|---|---|---|---|---|---|---|---|
| 200 | 800 | 115 | 1000 | 1000 | 2.75% | 9.9 | 101 |
| 20 | 980 | 1/50 | 1000 | 1000 | 5.00% | 12.0 | 83 |
| 200 | 800 | 115 | 1000 | 100 | 24.50% | 10.0 | 100 |
| 200 | 0 | neat | 1800 | 1000 | 2.50% | 102.0 | 10 |

### COMPARATIVE EXAMPLE 2

### Preparation of a Collagen Three-Dimensional Matrix

A high-surface-area, three-dimensional matrix can be made from reconstituted type I bovine collagen as set forth in O'Briena et ah, Biomaterials, 6: 1077-1086 (2004).. To determine if a matrix is suitable for enriching for cells, 5-40 mL samples of blood and various cell suspensions can be passed through the filter and processed as described in Example 1. Samples were successfully processed without noticeable clogging of commercially obtained collagen matrices, resulting in a depletion of approximately 85% of the erythrocytes and retention of almost 100% of the nucleated cells.

In enrichment experiments with heterogeneous mixtures of human nucleated cells, high-affinity, preferential attachment of stromal cells was attained compared to circulating leukocytic cells normally present in blood, without the use of capture antibodies.

### COMPARATIVE EXAMPLE 3

### Recovery of Ramos Tumor Cells

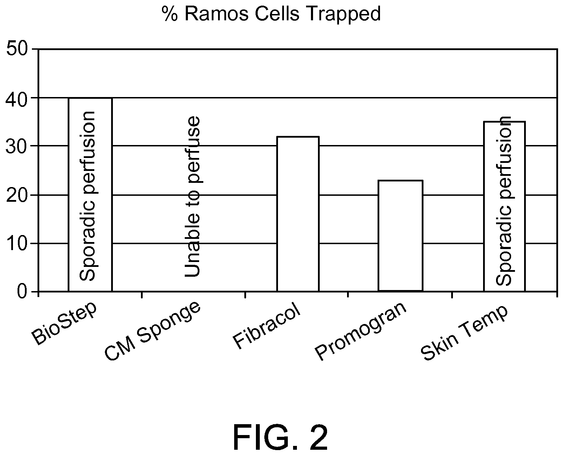

This examples describes the recovery of Ramos tumor cells using the following collagen matrices: BIOSTEP collagen matrix dressing (Smith & Nephew), Matrix Collagen Sponge^{™} wound dressing ("CM," Collagen Matrix, Inc.),
FIBRACOL collagen wound dressing (Johnson & Johnson), PROMOGRAN wound dressing (Allegro Medical), and SkinTemp collagen dressing (Medifil). One x 106 cells of Ramos tumor cells in suspension were loaded onto each matrix at 1 mL/minute. The collagen matrices were degraded using 2 U/ml collagenase, 100 U/ml dispase (Roche Industrial Enzymes). FIG. 2 is a graph that shows the percentage of total Ramos cells trapped using the matrices. The CM sponge was not suitable as it could not be perfused. While the Biostep and SkinTemp matrices retained a high percentage of tumor cells, perfusion was sporadic.

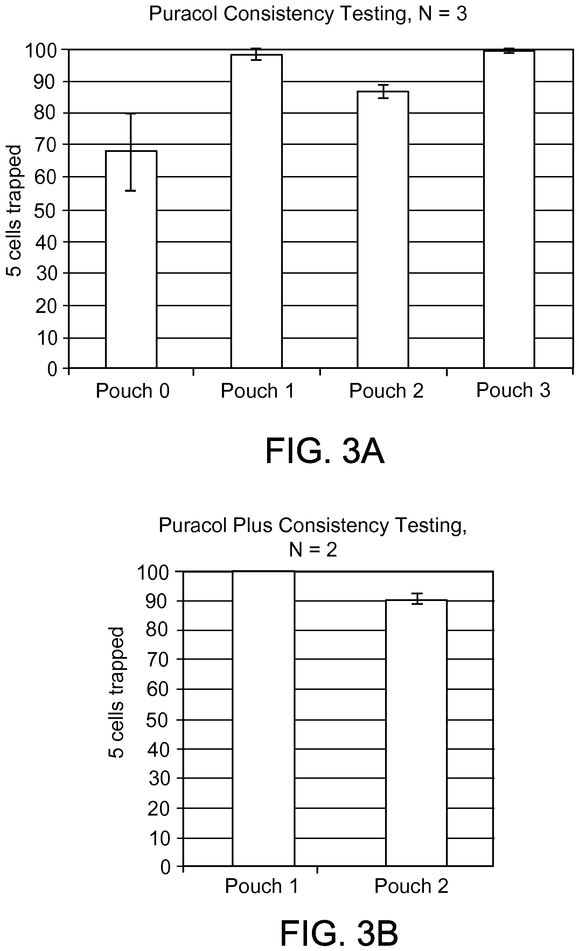

A similar experiment was repeated using the Ramos tumor cells and Puracol MicroScaffold^{™} collagen (Medline). FIG. 3 is a graph that shows the high percentage of trapped Ramos cells using the Purocol MicroScaffold^{™} collagen.

### COMPARATIVE EXAMPLE 4

### Recovery and Culture of Human Adipose Derived Stromal Cells (ADSCs)

Fresh human ADSCs were prepared from lipoaspirate by enzymatic digestion with a collagenase:dispase blend (2 U/ml collagenase, 100 U/ml dispase; Roche Industrial Enzymes) in Ringer's lactate, filtration through 100 µm mesh, and 3 × centrifugation and resuspension in cell growth media (MEM, 20 % FBS) at 105 nucleated cells/mL. One mL (105 cells) or 5 mL (5×l05 cells) cells were loaded onto a 1 cm diameter Puracol Plus collagen sponge by passage through a Swinnex filter assembly in which one or two 2 mm thick Puracol Plus disks had been placed. After loading, a sample of media that passed through the sponge was collected for cell count, and the remainder was centrifuged, resuspended in 2 mL growth media and plated (12 well plate). Loaded collagen disks were placed in 2 mL media and cultured in the same plate. Media was changed after 24h. Approximately 120h after initiation of culture, cells in monolayer (i.e., grown from the media that passed through the sponge) and cells on Puracol Plus disks were washed 3x with 2 mL Hank's Buffered Salt Solution (HBSS). Cells in monolayer were photographed under light microscope, detached by trypsinization with EDTA and counted. Cells on Puracol were released by digestion of Puracol for 30 min at 37°C with collagenase:dispase in Ringer's lactate. Cells were recovered by centrifugation and washed 3 x HBSS before resuspension in 2 mL culture media. Cell count was obtained, and then cells were plated onto plastic (12 well plate) and photographed approximately 6 h later. The results are depicted in Table 2.

**Table 2**

| **Condition** | **Cells not captured** | **Cell captured** | **Cells at 120h on plate** | **Cells at 120h in Puracol Plus** |
|---|---|---|---|---|
| 2 mm, 10⁵ cells | 62000 | 38000 | 210000 | 237500 |
| 2 mm, 5×10⁵ cells | 460000 | 40000 | 617500 | 225000 |
| 2x2 mm, 10⁵ cells | 0 | 100000 | 2500 | 175000 |
| 2x2 mm, 5×10⁵ cells | 230000 | 270000 | 397500 | 222500 |

### COMPARATIVE EXAMPLE 5

### Determining Degradation Time of Matrix

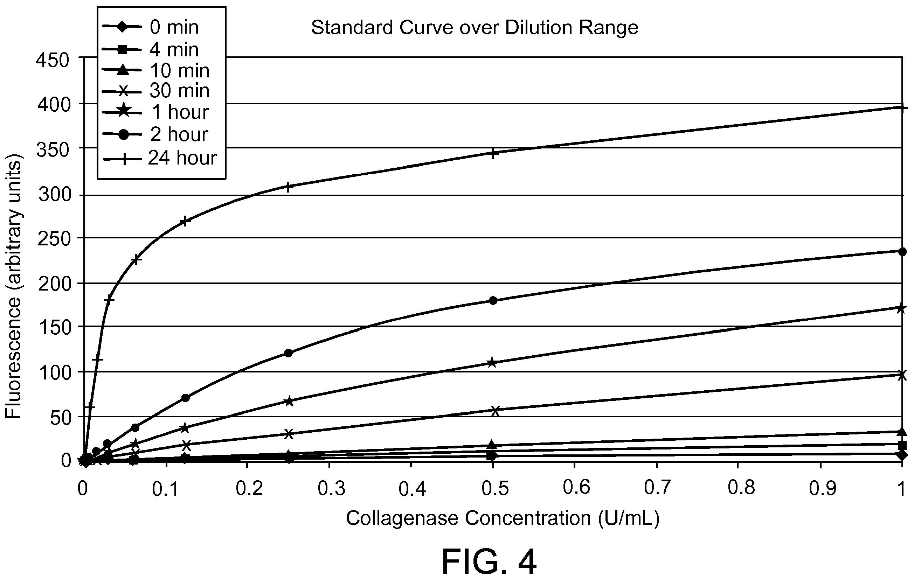

Titration curves of enzyme (e.g., collagenase or protease) concentration versus time can be developed for a given matrix as exemplified in FIG. 4 for gelatin beads (Cultispher) loaded with fluorescein. The fluorescence of the fluorescein is quenched within the beads. Upon digestion of the bead with the enzyme (e.g., a protease or collagenase), fluorescein is released.

## Claims

1. A method for obtaining target cells from a fluid, cell-containing sample, the method comprising:
a) providing a fluid, cell-containing sample;
b) passing the sample through a three-dimensional matrix, wherein the matrix comprises an inner core and an outer layer disposed around the inner core, the inner core comprising a nondegradable substrate, the outer layer composed of a degradable polymer and comprising a capture ligand attached thereto, wherein the capture ligand has affinity for a target cell in the sample; and
c) recovering target cells retained by said matrix by substantially degrading the outer layer of the matrix by contacting the matrix with a degradative enzyme.

2. The method of claim 1, wherein the fluid, cell-containing sample is peripheral blood, umbilical cord blood, bone marrow aspirate, lymph, cerebral spinal fluid, ductal fluid, needle biopsy aspirate, or a fluid portion of a lipoaspirate.

3. The method of claim 1 or 2, wherein the degradable polymer of the outer layer comprises an extracellular matrix molecule or product thereof.

4. The method of any one of claims 1 or 2, wherein the degradable polymer of the outer layer is composed of one or more natural or synthetic polymers.

5. The method of any one of claims 1 to 4, wherein the degradable polymer of the outer layer is selected from one or more of: poly(glycolic acid) (PGA), poly(lactic acid) (PLA), poly(lactic acid)-poly(glycolic acid) (PLGA), polyhydroxybutyrate (PHB), polyanhydride, polyorthoester, and poly(amino acid); or polycaprolactone.

6. The method of any one of claims 1 to 5, further comprising the step of cryopreserving the matrix and retained target cells prior to degrading the outer layer of the matrix.

7. The method of any one of claims 1 to 6, wherein the inner core is composed of a woven polypropylene, silk, mesh or fabric.

8. The method of claim 7, wherein the inner core is composed of a non-woven polypropylene, silk, mesh or fabric.

9. The method of any one of claims 1 - 8, wherein the fluid, cell-containing sample is first centrifuged to fractionate cells according to their respective specific gravity before passing one or more of the cell fractions through the three dimensional matrix.

10. The method of claim 1 or 2, wherein the outer layer comprises a polysaccharide.

## Patentansprüche

1. Verfahren zur Gewinnung von Zielzellen aus einer fluiden, zellhaltigen Probe, wobei das Verfahren umfasst:
a) Bereitstellen einer fluiden, zellhaltigen Probe;
b) Durchleiten der Probe durch eine dreidimensionale Matrix, wobei die Matrix einen inneren Kern und eine um den inneren Kern herum angeordnete äußere Schicht umfasst, wobei der innere Kern ein nicht abbaubares Substrat umfasst, die äußere Schicht aus einem abbaubaren Polymer besteht und einen daran befestigten Einfangliganden umfasst, wobei der Einfangligand eine Affinität für eine Zielzelle in der Probe aufweist; und
c) Gewinnen von Zielzellen, die von der Matrix zurückgehalten werden, indem die äußere Schicht der Matrix im Wesentlichen abgebaut wird, indem die Matrix mit einem abbauenden Enzym in Kontakt gebracht wird.

2. Verfahren nach Anspruch 1, wobei die fluide, zellhaltige Probe peripheres Blut, Nabelschnurblut, Knochenmarkaspirat, Lymphe, zerebrales Rückenmarksfluid, Ductusfluid, Nadelbiopsie-Aspirat oder einen fluiden Teil eines Lipoaspirats ist.

3. Verfahren nach Anspruch 1 oder 2, wobei das abbaubare Polymer der äußeren Schicht ein extrazelluläres Matrixmolekül oder Produkt davon umfasst.

4. Verfahren nach einem beliebigen der Ansprüche 1 oder 2, wobei das abbaubare Polymer der äußeren Schicht aus einem oder mehreren natürlichen oder synthetischen Polymeren zusammengesetzt ist.

5. Verfahren nach einem beliebigen der Ansprüche 1 bis 4, wobei das abbaubare Polymer der äußeren Schicht ausgewählt ist aus einem oder mehreren der folgenden Polymere: Poly(glykolsäure) (PGA), Poly(milchsäure) (PLA), Poly(milchsäure)-poly(glykolsäure) (PLGA), Polyhydroxybutyrat (PHB), Polyanhydrid, Polyorthoester und Poly(aminosäure); oder Polycaprolacton.

6. Verfahren nach einem beliebigen der Ansprüche 1 bis 5, ferner umfassend den Schritt von Kryokonservierung der Matrix und der zurückgehaltenen Zielzellen vor Abbau der äußeren Schicht der Matrix.

7. Verfahren nach einem beliebigen der Ansprüche 1 bis 6, wobei der innere Kern aus einem gewebten Polypropylen, Seide, Netz oder Gewebe besteht.

8. Verfahren nach Anspruch 7, wobei der innere Kern aus einem nichtgewebten Polypropylen, Seide, Netz oder Gewebe besteht.

9. Verfahren nach einem beliebigen der Ansprüche 1 bis 8, wobei die fluide, zellhaltige Probe zuerst zentrifugiert wird, um die Zellen entsprechend ihrem jeweiligen spezifischen Gewicht zu fraktionieren, bevor eine oder mehrere der Zellfraktionen durch die dreidimensionale Matrix geleitet werden.

10. Verfahren nach Anspruch 1 oder 2, wobei die äußere Schicht ein Polysaccharid umfasst.

## Revendications

1. Procédé d'obtention de cellules cibles à partir d'un échantillon fluide contenant des cellules, le procédé comprenant :
a) la fourniture d'un échantillon fluide contenant des cellules ;
b) le passage de l'échantillon à travers une matrice tridimensionnelle, dans lequel la matrice comprend un noyau interne et une couche externe disposée autour du noyau interne, le noyau interne comprenant un substrat non dégradable, la couche externe étant composée d'un polymère dégradable et comprenant un ligand de capture fixé à celui-ci, dans lequel le ligand de capture a une affinité pour une cellule cible dans l'échantillon ; et
c) la récupération de cellules cibles retenues par ladite matrice par dégradation substantielle de la couche externe de la matrice par mise en contact de la matrice avec une enzyme de dégradation.

2. Procédé selon la revendication 1, dans lequel l'échantillon fluide contenant des cellules est du sang périphérique, du sang de cordon ombilical, une aspiration de moelle osseuse, de la lymphe, du liquide céphalo-rachidien, du fluide canalaire, une aspiration de biopsie à l'aiguille ou une partie fluide d'une lipoaspiration.

3. Procédé selon la revendication 1 ou 2, dans lequel le polymère dégradable de la couche externe comprend une molécule de matrice extracellulaire ou un produit de celle-ci.

4. Procédé selon l'une quelconque des revendications 1 ou 2, dans lequel le polymère dégradable de la couche externe est composé d'un ou plusieurs polymères naturels ou synthétiques.

5. Procédé selon l'une quelconque des revendications 1 à 4, dans lequel le polymère dégradable de la couche externe est choisi parmi un ou plusieurs de : poly(acide glycolique) (PGA), poly(acide lactique) (PLA), poly(acide lactique)-poly(acide glycolique) (PLGA), polyhydroxybutyrate (PHB), polyanhydride, polyorthoester et poly(acide aminé) ; ou polycaprolactone.

6. Procédé selon l'une quelconque des revendications 1 à 5, comprenant en outre l'étape de cryoconservation de la matrice et des cellules cibles retenues avant la dégradation de la couche externe de la matrice.

7. Procédé selon l'une quelconque des revendications 1 à 6, dans lequel le noyau interne est composé de polypropylène, soie, maille ou tissu tissé.

8. Procédé selon la revendication 7, dans lequel le noyau interne est composé de polypropylène, soie, maille ou tissu non tissé.

9. Procédé selon l'une quelconque des revendications 1 à 8, dans lequel l'échantillon fluide contenant des cellules est dans un premier temps centrifugé pour fractionner les cellules en fonction de leur densité avant de faire passer une ou plusieurs des fractions de cellules à travers la matrice tridimensionnelle.

10. Procédé selon la revendication 1 ou 2, dans lequel la couche externe comprend un polysaccharide.
